# FASCICULE DE BREVET EUROPEEN

(11) **EP 1 138 703 B1**
(45) Date de publication et mention de la délivrance du brevet: **20.07.2005**
(21) Numéro de dépôt: 01400635.7
(22) Date de dépôt: 09.03.2001
(51) Int. Cl.: C08F 220/18, A61K 7/48

(54) **Composition cosmétique**
Kosmetische Zusammensetzung
Cosmetic composition

(30) Priorité: 29.03.2000 FR 0003955
(43) Date de publication de la demande: 04.10.2001
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Mallo, Paul, 78400 Chatou (FR); Tabacchi, Guy, 81100 Castres (FR); Brancq, Bernard, 78150 Le Chesnay (FR); Boiteux, Jean-Pierre, 81710 Saix (FR)
(74) Mandataire: Conan, Philippe Claude

(56) Documents cités:
- EP-A- 0 562 344
- DE-A- 2 638 386
- US-A- 3 030 347
- US-A- 4 147 850

## Description

La présente demande se rapporte à une nouvelle composition topique cosmétique, dermocosmétique, dermopharmaceutique ou pharmaceutique contenant des polymères épaississants et/ou émulsionnants.

Différents copolymères émulsionnants constitués d'une fraction majoritaire d'un monomère acide carboxylique insaturé comportant de trois à six atomes de carbone ou de son anhydride, et d'une fraction minoritaire d'un monomère ester gras d'acide acrylique, sont commercialisés sous le nom de PEMULEN™. Ils sont décrits dans les brevets américains 5,373,044 et 5,288,814. Cependant, ces polymères doivent être neutralisés pour devenir émulsionnants et ils ne fonctionnent donc pas en milieu acide. Les poly(2-acrylamido-2-méthyl propanesulfonates de métaux alcalins) sont décrits dans la demande de brevet européen publiée sous le numéro 0 814 403. De tels polymères ne présentent cependant pas de propriétés émulsionnantes. La demande de brevet européen EP 0562 344 divulgue un tétrapolymère de d'acide 2-acrylamido-2-méthyl propanesulfonique, d'acide acrylique, d'acide méthacrylique et d'acrylate d'éthyl hexyle utilisé comme colloïde protecteur. La demanderesse s'est donc intéressée à la synthèse et à la mise au point de polymères épaississants et émulsionnants, sous forme solide, prêts à l'emploi, c'est à dire ne nécessitant pas de neutralisation, pouvant être facilement dispersés dans l'eau et pouvant être utilisés sur une large gamme de pH.

L'invention a pour objet une composition topique cosmétique, dermocosmétique, dermopharmaceutique ou pharmaceutique, caractérisée en ce qu'elle comprend de 0,1 % à 10 % en poids d'un polymère, linéaire, branché ou réticulé, à base d'au moins un monomère possédant une fonction acide fort libre, partiellement salifiée ou totalement salifiée, copolymérisé avec au moins un monomère choisi, soit parmi les esters d'alcools aliphatiques comportant de 8 à 30 atomes de carbone et d'acides monocarboxyliques insaturés, soit parmi les esters d'alcools aliphatiques comportant de 8 à 30 atomes de carbone et d'acides polycarboxyliques insaturés.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention peut comporter des motifs réticulés et/ou des motifs branchés.

L'agent de réticulation et/ou l'agent de ramification est choisi parmi les monomères polyéthylèniques, tels que par exemple, l'acide diallyloxyacétique, CH(O-CH₂-CH=CH₂)₂-C(=O)-OH ou un des sels et notamment son sel de sodium, le triméthylolpropanetriacrylate, CH₃-C[CH₂-O-C(=O)-CH=CH₂]₃, le diméthacrylate d'éthylèneglycol, CH₂=C(CH₃)-C(=O)-O-(CH₂)₂-O-C(=O)-C(CH₃)=CH₂, le diacrylate de diéthylèneglycol, CH₂=CH-C(=O)-O-[(CH₂)₂-O]₂-C(=O)-CH=CH₂, le diacrylate de tétraéthylèneglycol, CH₂=CH-C(=O)-O-[(CH₂)₂-O-]₄-C(=O)-CH=CH₂, le méthylène bis(acrylamide), CH₂=CH-C(=O)-NH-CH₂-NH-C(=O)-CH=CH₂, le diallyl urée, CH₂=CH-CH₂-NH-C(=O)-NH-CH₂-CH=CH₂, le triallylamine, N(-CH₂-CH=CH₂)₃ ou le 1,1,2,2-tétrallyloxy éthane (tétraallyloxyéthane), CH(O-CH₂-CH=CH₂)₂-CH(O-CH₂-CH=CH₂)₂

Par partiellement ou totalement salifiée, on signifie que les fonctions acides forts sont partiellement ou totalement salifiées sous forme, notamment de sel de métal alcalin, tel que le sel de sodium ou le sel de potassium, de sel d'ammonium (NH₄⁺) ou de sel d'aminoalcool, tel que par exemple, le sel de monoéthanolamine (HOCH₂CH₂NH₃⁺).

La fonction acide fort du monomère en comportant est notamment la fonction acide sulfonique ou la fonction acide phosphonique. Ledit monomère peut être par exemple l'acide styrènesulfonique, le méthacrylate de (2-sulfo éthyle), l'acide styrènephosphonique ou, de préférence, l'acide 2-méthyl 2-[(1-oxo 2-propènyl) amino] 1-propanesulfonique, CH₃-C(CH₃)[NH-C(=O)-CH=CH₂]-CH₂-SO₃H, lesdits acides étant libres, partiellement salifiés ou totalement salifiés.

Par esters d'acides polycarboxyliques insaturés, on désigne les monoesters ou les polyesters desdits acides. Ainsi, lorsqu'il s'agit d'un diacide, la définition inclut le monoester et le diester. L'acide carboxylique insaturé est plus particulièrement choisi parmi les acides α-insaturés, tels que les mono acides carboxyliques α-insaturés ou les diacides carboxyliques α-insaturés, comme par exemple,
l'acide acrylique, CH₂=CH-C(=O)-OH,
l'acide méthacrylique, CH₂=C(CH₃)-C(=O)-OH,
l'acide itaconique, CH₂[C(=O)-OH]-CH(=CH₂)-C(=O)-OH, ou
l'acide maléique, CH[C(=O)-OH]=CH-C(=O)-OH.

Par radical aliphatique comportant de 8 à 30 atomes de carbone on désigne plus particulièrement un radical saturé ou insaturé, linéaire ou ramifié.

Par radical aliphatique hydrocarboné linéaire et saturé ou insaturé, comprenant de 8 à 30 atomes de carbone, on désigne plus particulièrement, les radicaux dérivés des alcools primaires linéaires tels que par exemple, ceux dérivés des alcools octylique, pélargonique, décylique, undécylique, undécénylique, laurique, tridécylique, myristylique, pentadécylique, cétylique, heptadécylique, stéarylique, oléylique, linoléylique, nonadécylique, arachidique, béhènylique, érucylique ou 1-triacontanoïque. Il s'agit alors des radicaux octyle, nonyle, décyle, undécyle, 10-undécènyle, dodécyle, tridécyle, tétradécyle, pentadécyle, hexadécyle, heptadécyle, octadécyle, 9-octadécènyle, 10,12-octadécadiènyle, nonadécyle, 13-docosènyle, docosanyle ou triacontanyle.

Par radical hydrocarboné ramifié et saturé comprenant de 8 à 30 atomes de carbone, on désigne plus particulièrement, soit les radicaux dérivés des alcools de Guerbet, qui sont des 1-alcanols ramifiés répondant à la formule générale :

CH₃-(CH₂)ₚ-CH[CH₃-(CH₂)ₚ₋₂]-CH₂OH,

dans laquelle p représente un nombre entier compris entre 2 et 14, tels que, par exemple, les radicaux 2-éthyl hexyle, 2-propyl heptyle, 2-butyl octyle, 2-pentyl nonyle, 2-hexyl décyle ou 2-octyl dodécyle, soit les radicaux dérivés des isoalcanols répondant à la formule générale:

CH₃-CH(CH₃)-(CH₂)ₘ-CH₂OH,

dans laquelle m représente un nombre entier compris entre 2 et 26 tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle.

L'invention a aussi pour objet une composition telle que définie précédemment, dans laquelle 30% à 98% des motifs monomériques que ledit polymère comprend, possèdent une fonction acide fort libre, partiellement ou totalement salifiée.

Le polymère tel de défini précédemment, contient plus particulièrement de 30% à 98% en poids d'au moins un monomère possédant un fonction acide fort, de 1% à 15% en poids d'au moins un ester à chaîne grasse d'un acide carboxylique insaturé, de 0,01% à 5% en poids d'au moins un monomère polyéthylènique et de 0% à 60% en poids d'au moins un monomère n'appartenant pas aux familles définies ci-dessus, comme par exemple, un monomère neutre ou un monomère à fonction acide carboxylique non estérifiée, libre, partiellement salifiée ou totalement salifiée.

Comme monomère neutre, il y a par exemple, l'acrylamide, CH₂=CH-C(=O)-NH, le méthacrylamide, CH₂=C(CH₃)-C(=O)-NH, le vinyl pyrrolidone, 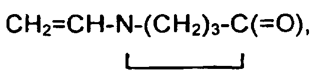 l'acrylate de (2-hydroxy éthyle), CH₂=CH-C(=O)-O-CH₂-CH₂-OH, le méthacrylate de (2-hydroxy éthyle), CH₂=C(CH₃)-C(=O)-O-CH₂-CH₂-OH, l'acrylate de (2,3-dihydroxy propyle), CH₂=CH-C(=O)-O-CH₂-CH(OH)-CH₂-OH, le méthacrylate de (2,3-dihydroxy propyle) CH₂=C(CH₃)-C(=O)-O-CH₂-CH(OH)-CH₂-OH ou un dérivé éthoxylé de poids moléculaire compris entre 400 et 1000, de chacun des esters hydroxylés décrits ci-dessus.

Comme monomère à fonction acide carboxylique non estérifiée, il y a, par exemple, l'acide acrylique, l'acide méthacrylique, l'acide itaconique ou l'acide maléique. Lorsque ces acides sont partiellement ou totalement salifiés, il s'agit plus particulièrement de sel de métal alcalin, tel que le sel de sodium ou le sel de potassium, de sel d'ammonium (NH₄⁺) ou de sel d'aminoalcool, tel que par exemple, le sel de monoéthanolamine (HOCH₂CH₂NH₃⁺).

Selon un autre aspect de la présente invention, celle-ci a pour objet un procédé de préparation du polymère tel que défini précédemment, caractérisé en ce que :
a) l'on dissout l'ensemble des monomères dans un solvant,
b) l'on amorce la réaction de polymérisation par introduction dans la solution formée en a), d'un initiateur de radicaux libres puis on la laisse se dérouler,
c) lorsque la réaction de polymérisation est terminée, on filtre et sèche le résidu obtenu pour obtenir ledit polymère sous forme solide.

Le solvant dans lequel est effectuée la dissolution des monomères est choisi, par exemple, parmi le cyclohexane, l'acétate d'éthyle ou le tert-butanol. La réaction de polymérisation est initiée à l'aide d'un amorceur peroxydique ou azoïque, tel que par exemple, le dilaurylperoxyde, l'azo bis(isobutyronitrile) (AIBN) ou le diperoxydicarbonate de bis(2-éthyl hexyle). La réaction de polymérisation est conduite entre 35 et 85°C, plus particulièrement entre 50 et 70°C, sous pression atmosphérique et pendant environ 2 heures.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau et des muqueuses. La composition topique comporte éventuellement un principe actif qui peut, par exemple, consister en un agent anti-inflammatoire, un myorelaxant, un antifongique ou un antibactérien.

Lorsque la composition topique est utilisée en tant que composition cosmétique destinée à être appliquée sur la peau ou les muqueuses, elle peut ou non comporter un principe actif, par exemple un agent hydratant, un agent bronzant, un filtre solaire, un antirides, un agent à visée amincissante, un agent antiradicalaire, un agent antiacnéique ou un antifongique.

Le pH de la composition topique est de préférence, supérieur ou égal à 5.

La composition topique peut en outre comporter des composés classiquement compris dans ce type de compositions, par exemple des parfums, des conservateurs, des colorants, des émollients ou des tensioactifs.

Les exemples suivants illustrent la présente invention, sans toutefois la limiter.

### Exemple 1: Copolymère AMPS/MAC₁₈ester (97/3) réticulé au TAOE

On charge dans un réacteur successivement et sous agitation :
- 1000 g de tertio-butanol,
- 6,75 g de méthacrylate d'octadécyle commercial (MA C₁₈ester),
- 150 g d'acide 2-méthyl 2-[(1-oxo 2-propényl) amino] 1-propanesulfonique commercial (AMPS™),
- 12,3 g d'ammoniac,
- 4 g de tétraallyloxyéthane (TAOE)

Le mélange est soumis à un barbotage d'azote pendant environ 1 heure à température ambiante puis il est porté à 70°C, température à laquelle on y introduit 0,3 g de peroxydicarbonate de bis(2-éthyl hexyle). Il est ensuite maintenu pendant environ 2 heures à cette température puis il est laissé au reflux pendant deux heures. Après filtration, et séchage sous pression réduite à 60°C pendant 8 heures, on obtient le copolymère AMPS/MAC₁₈ester, réticulé au tétraallyloxyé thane, dans lequel le ratio molaire en monomères, AMPS/MAC₁₈ester, est égal à 97/3.

### Exemple 2: Copolymère AMPS/MAC₁₈ester (94/6) réticulé au TAOE

Le composé est obtenu en opérant de la même manière qu'à l'exemple 1, mais en utilisant 13 g de méthacrylate d'octadécyle et 146 g d'acide 2-methyl-2-[(1-oxo 2-propènyl) amino] 1-propanesulfonique, pour que le ratio molaire en monomères AMPS/MAC₁₈ester soit égal à 94/6.

### Exemple 3: Copolymère AMPS/MAC₁₈ester (97/3) réticulé au TMPTA

Le composé est obtenu en opérant de la même manière qu'à l'exemple 1, mais en utilisant 4g triméthylolpropanetricacrylate (TMPTA), à la place du tétraallyloxyéthane.

### Exemple 4: Copolymère AMPS/ MAC₁₈ester (88/12) réticulé au TAOE

On charge dans un réacteur successivement et sous agitation :
- 1000 g de tertio-butanol,
- 26 g de méthacrylate d'octadécyle
- 136,8 g d'acide 2-méthyl 2-[(1-oxo 2-propényl) amino] 1-propane sulfonique commercial (AMPS™),
- 11 g d'ammoniac,
- 3 g de tétraallyloxyéthane (TAOE) Le mélange est soumis à un barbotage d'azote pendant environ 1 heure à température ambiante puis il est porté à 70°C température à laquelle On y introduit 4 g de d'azo bis(isobutyronitrile). Il est en suite maintenu environ 2 heures à cette température puis il est laissé au reflux pendant deux heures. Après filtration, et séchage sous pression réduite à 70°C pendant 8 heures, on obtient le produit attendu.

### Exemple 5 : Copolymère AMPS/AM/ MAC₁₈ester (64/37/3) réticulé au MBA

On charge dans un réacteur successivement et sous agitation :
- 1000 g de tertio-butanol,
- 6,75 g de méthacrylate d'octadécyle,
- 100 g d'acide 2-méthyl 2-[(1-oxo 2-propényl) amino] 1-propanesulfonique commercial (AMPS™),
- 8,2 g d'ammoniac,
- 127,57,2 g d'acrylamide (AM),
- 0,5 g de méthylène bis(acrylamide (MBA).

Le mélange est soumis à un barbotage d'azote pendant environ 1 heure à température ambiante puis il porté à 70°C, température à laquelle on y introduit 1,0 g de peroxyde de dilauryle. Il est ensuite maintenu environ 2 heures à cette température puis il est laissé au reflux pendant deux heures. Après filtration, et séchage sous pression réduite à 60°C pendant 8 heures, on obtient le terpolymère attendu.

### Exemple 6: Copolymère AM/AMPS/ MAC₁₈ester (52/45/3) réticulé au MBA

Le composé est obtenu en opérant de la même manière qu'à l'exemple 4, mais en utilisant 6,75 g de méthacrylate d'octadécyle, 70 g d'acide 2-methyl-2-[(1-oxo 2-propènyl) amino] 1-propanesulfonique, 27,5 g d'acrylamide et 5,75 g d'ammoniaque.

### Exemple 7: Copolymère AMPSIAMIHEAI MAC₁₈ester (64/27/10/3) réticulé au MBA

Le composé est obtenu en opérant de la même manière qu'à l'exemple 4, mais en remplaçant les 17,2 g d'acrylamide, par 12,5 g d'acrylamide et 7,65 g d'acrylate d'hydroxyéthyle (HEA).

### Exemple 8: Copolymère AMPS/MAC₁₂ester (97/3) réticulé au TAOE

Le composé est obtenu en opérant de la même manière qu'à l'exemple 1, mais en remplaçant le méthacrylate d'octadécyle, par une quantité équimolaire de méthacrylate de lauryle (MAC₁₂ester).

### Exemple 9 : Copolymère AMPS/AAC₁₆ester (97/3) réticulé au TAOE

Le composé est obtenu en opérant de la même manière qu'à l'exemple 1, mais en remplaçant le méthacrylate d'octadécyle, par une quantité équimolaire d'acrylate d'hexadécyle (AAC₁₆ester).

### Exemple 10 : Copolymère AMPS/MAC₈ ester (97/3) réticulé au TAOE

Le composé est obtenu en opérant de la même manière qu'à l'exemple 1, mais en remplaçant le méthacrylate d'octadécyle, par une quantité équimolaire de méthacrylate d'octyle (MAC₈ester).

### Exemple 11 : Copolymère AMPS/MAC₃₀ester (88/12) réticulé au TAOE

Le composé est obtenu en opérant de la même manière qu'à l'exemple 4, mais en remplaçant le méthacrylate d'octadécyle par une quantité équimolaire de méthacrylate de n-triacontanyle (MAC₃₀ester) et en ajustant la température de réaction à 85°C.

### Exemple 12 : Copolymère AMPS/AAC₂₂ester (97/3) non réticulé

Le composé est obtenu en opérant de la même manière qu'à l'exemple 1, mais en l'absence de tétraallyloxyéthane, en remplaçant le méthacrylate d'octadécyle par une quantité équimolaire d'acrylate de docosanyle (AAC₂₂ester) et en ajustant la température de réaction à 80°C.

## Revendications

1. Composition topique cosmétique, dermocosmétique, dermopharmaceutique ou pharmaceutique, **caractérisée en ce qu'**elle comprend de 0,1 % à 10 % en poids d'un polymère linéaire, branché ou réticulé, à base d'au moins un monomère possédant une fonction acide fort libre, partiellement salifiée ou totalement salifiée, copolymérisé avec au moins un monomère choisi, soit parmi les esters d'alcools aliphatiques comportant de 8 à 30 atomes de carbone et d'acides monocarboxyliques insaturés, soit parmi les esters d'alcools aliphatiques comportant de 8 à 30 atomes de carbone et d'acides polycarboxyliques insaturés.

2. Composition telle que définie à la revendication 1, dans laquelle ledit polymère est branché ou réticulé avec un agent de réticulation et/ou l'agent de ramification choisi parmi l'acide diallyloxyacétique ou un des sels et notamment son sel de sodium, le triméthylolpropanetriacrylate, le diméthacrylate d'éthylène-glycol, le diacrylate de diéthylèneglycol, le diacrylate de tétraéthylèneglycol, le méthylène bis (acrylamide), le diallyl urée, le triallylamine ou le 1,1,2,2-tétrallyloxy éthane.

3. Composition telle que définie à l'une des revendications 1 ou 2, pour laquelle le monomère à fonction acide fort dudit polymère est partiellement ou totalement salifiée sous forme de sel de métal alcalin, tel que le sel de sodium ou le sel de potassium, de sel d'ammonium (NH₄⁺) ou de sel d'aminoalcool, tel que par exemple, le sel de monoéthanolamine (HOCH₂CH₂NH₃⁺).

4. Composition telle que définie à l'une des revendications 1 à 3, pour laquelle la fonction acide fort du monomère dudit polymère en comportant, est la fonction acide sulfonique ou la fonction acide phosphonique.

5. Composition telle que définie à l'une des revendications 1 à 4, pour laquelle le monomère à fonction acide fort dudit polymère est choisi parmi l'acide styrènesulfonique, l'acide styrènephosphonique, le méthacrylate de (2-sulfo éthyle) ou de préférence l'acide 2-méthyl 2-[(1-oxo 2-propènyl) amino] 1-propanesulfonique, lesdits acides étant libres, partiellement salifiés ou totalement salifiés.

6. Composition telle que définie à l'une des revendications 1 à 5, pour laquelle les esters d'alcools aliphatiques comportant de 8 à 30 atomes de carbone et d'acides monocarboxyliques ou polycarboxyliques insaturés dudit polymère, sont choisis parmi les esters d'acides α-insaturés et plus particulièrement parmi les mono acides carboxyliques α-insaturés ou les diacides carboxyliques α-insaturés.

7. Composition telle que définie à la revendication 6, pour laquelle les esters de monoacides carboxyliques α-insaturés ou de diacides carboxyliques α-insaturés dudit polymère, sont choisis parmi les esters de l'acide acrylique, de l'acide méthacrylique, de l'acide itaconique ou de l'acide maléique.

8. Composition telle que définie à l'une des revendications 1 à 7, pour laquelle le radical aliphatique comportant de 8 à 30 atomes de carbone de la fonction ester dudit polymère, est un radical saturé ou insaturé, linéaire ou ramifié.

9. Composition telle que définie à la revendication 8, pour laquelle le radical aliphatique comportant de 8 à 30 atomes de carbone de la fonction ester dudit polymère, est choisi parmi les radicaux dérivés des alcools primaires linéaires et plus particulièrement parmi les radicaux octyle, nonyle, décyle, undécyle, 10-undécènyle, dodécyle, tridécyle, tétradécyle, pentadécyle, hexadécyle, heptadécyle, octadécyle, 9-octadécènyle, 10,12-octadécadiènyle, 13-docosènyle ou triacontanyle.

10. Composition telle que définie à la revendication 8, pour laquelle le radical aliphatique comportant de 8 à 30 atomes de carbone de la fonction ester dudit polymère, est choisi parmi les radicaux dérivés des 1-alcanols ramifiés répondant à la formule générale:
CH₃-(CH₂)ₚ-CH[CH₃-(CH₂)ₚ₋₂]-CH₂OH,
dans laquelle p représente un nombre entier compris entre 2 et 14, et plus particulièrement parmi les radicaux 2-éthyl hexyle, 2-propyl heptyle, 2-butyl octyle, 2-pentyl nonyle, 2-hexyl décyle ou 2-octyl dodécyle.

11. Composition telle que définie à la revendication 8, pour laquelle le radical aliphatique comportant de 8 à 30 atomes de carbone de la fonction ester dudit polymère, est choisi parmi les radicaux 2-hexyl octyle, 2-octyl décyle ou 2-hexyl dodécyle ou parmi les radicaux dérivés des isoalcanols répondant à la formule générale :
CH₃-CH(CH₃)-(CH₂)ₘ-CH₂OH,
dans laquelle m représente un nombre entier compris entre 2 et 26 et plus particulièrement parmi les radicaux 4-méthyl pentyle, 5-méthyl hexyle, 6-méthyl heptyle, 15-méthyl pendadécyle ou 16-méthyl heptadécyle.

12. Composition telle que définie à l'une des revendications 1 à 11, pour laquelle 30% à 98% des motifs monomériques que ledit polymère comprend, possèdent une fonction acide fort libre, partiellement salifiée ou totalement salifiée.

13. Composition telle que définie à la revendication 12, dans laquelle ledit polymère comporte de 30% à 98% d'au moins un monomère possédant une fonction acide fort, de 1% à 15% d'au moins un ester à chaîne grasse d'un acide carboxylique insaturé, de 0,01% à 5% d'au moins un monomère polyéthylènique et de 0% à 60% en poids d'au moins un monomère choisi parmi les monomères neutres ou les monomère à fonction acide carboxylique non estérifiée, libre partiellement salifiée ou totalement salifiée.

14. Composition telle que définie à la revendication 13, pour laquelle le monomère neutre dudit polymère en comportant éventuellement, est choisi parmi l'acrylamide, le méthacrylamide, le vinyl pyrrolidone, l'acrylate de (2-hydroxy éthyle), le méthacrylate de (2-hydroxy éthyle), l'acrylate de (2,3-dihydroxy propyle), le méthacrylate de (2,3-dihydroxy propyle) ou un dérivé éthoxylé de poids moléculaire compris entre 400 et 1000, de chacun des esters hydroxylés décrits ci-dessus et le monomère à fonction acide carboxylique non estérifiée est choisi parmi l'acide acrylique, l'acide méthacrylique, l'acide itaconique ou l'acide maléique.

## Patentansprüche

1. Kosmetische, dermokosmetische, dermopharmazeutische oder pharmazeutische topische Zusammensetzung, **dadurch gekennzeichnet, daß** sie 0,1 bis 10 Gew.-% eines linearen, verzweigten oder vernetzten Polymers auf Basis mindestens eines Monomers mit einer freien teilweise oder vollständig versalzten starken Säurefunktion, das mit mindestens einem entweder unter Estern von aliphatischen Alkoholen mit 8 bis 30 Kohlenstoffatomen und ungesättigten Monocarbonsäuren oder unter Estern von aliphatischen Alkoholen mit 8 bis 30 Kohlenstoffatomen und ungesättigten Polycarbonsäuren ausgewählten Monomer copolymerisiert ist.

2. Zusammensetzung nach Anspruch 1, bei der das Polymer mit einem unter Diallyloxyessigsäure oder einem der Salze, insbesondere dem Natriumsalz davon, Trimethylpropantriacrylat, Ethylenglykoldimethacrylat, Diethylenglykoldiacrylat, Tetraethylenglykoldiacrylat, Methylenbis(acrylamid), Diallylharnstoff, Triallylamin oder 1,1,2,2-Tetraallyloxyethan ausgewählten Vernetzungsmittel und/oder Verzweigungsmittel verzweigt oder vernetzt ist.

3. Zusammensetzung nach Anspruch 1 oder 2, bei der das eine starke Säurefunktion enthaltende Monomer des Polymers teilweise oder vollständig in Form eines Alkalimetallsalzes, wie des Natriumsalzes oder des Kaliumsalzes, eines Ammoniumsalzes (NH₄⁺) oder Aminoalkoholsalzes, wie beispielsweise des Monoethanolaminsalzes (HOCH₂CH₂NH₃⁺), versalzt ist.

4. Zusammensetzung nach einem der Ansprüche 1 bis 3, bei der es sich bei der starken Säurefunktion des Monomers des dieses enthaltenden Polymers um die Sulfonsäurefunktion oder die Phosphonsäurefunktion handelt.

5. Zusammensetzung nach einem der Ansprüche 1 bis 4, bei der das eine starke Säurefunktion enthaltende Monomer des Polymers unter Styrolsulfonsäure, Styrolphosphonsäure, (2-Sulfoethyl)methacrylat oder vorzugsweise 2-Methyl-2-[(1-oxo-2-propenyl)-amino]-1-propansulfonsäure ausgewählt ist, wobei diese Säuren frei, teilweise versalzt oder vollständig versalzt sind.

6. Zusammensetzung nach einem der Ansprüche 1 bis 5, bei der die Ester von aliphatischen Alkoholen mit 8 bis 30 Kohlenstoffatomen und ungesättigten Mono- oder Polycarbonsäuren des Polymers unter Estern von α-ungesättigten Säuren und insbesondere unter α-ungesättigten Monocarbonsäuren oder α-ungesättigten Dicarbonsäuren ausgewählt sind.

7. Zusammensetzung nach Anspruch 6, bei der die Ester von α-ungesättigten Monocarbonsäuren oder α-ungesättigten Dicarbonsäuren des Polymers unter Estern von Acrylsäure, Methacrylsäure, Itaconsäure oder Maleinsäure ausgewählt sind.

8. Zusammensetzung nach einem der Ansprüche 1 bis 7, bei der es sich bei dem 8 bis 30 Kohlenstoffatome enthaltenden aliphatischen Rest der Esterfunktion des Polymers um einen linearen oder verzweigten, gesättigten oder ungesättigten Rest handelt.

9. Zusammensetzung nach Anspruch 8, bei der der 8 bis 30 Kohlenstoffatome enthaltende aliphatische Rest der Esterfunktion des Polymers unter Resten, die sich von linearen primären Alkoholen ableiten, und insbesondere unter Octyl-, Nonyl-, Decyl-, Undecyl-, 10-Undecenyl-, Dodecyl-, Tridecyl-, Tetradecyl-, Pentadecyl-, Hexadecyl-, Heptadecyl-, Octadecyl-, 9-Octadecenyl-, 10,12-Octadecadienyl-, 13-Docosenyl- oder Triacontanylresten ausgewählt ist.

10. Zusammensetzung nach Anspruch 8, bei der der 8 bis 30 Kohlenstoffatome enthaltende aliphatische Rest der Esterfunktion des Polymers unter Resten, die sich von verzweigten 1-Alkanolen der allgemeinen Formel
CH₃-(CH₂)ₚ-CH[CH₃-(CH₂)ₚ₋₂]-CH₂OH,
worin p für eine ganze Zahl zwischen 2 und 14 steht, ableiten, und insbesondere unter 2-Ethylhexyl-, 2-Propylheptyl-, 2-Butyloctyl-, 2-Pentylnonyl-, 2-Hexyldecyl- oder 2-Octyldodecylresten ausgewählt ist.

11. Zusammensetzung nach Anspruch 8, bei der der 8 bis 30 Kohlenstoffatome enthaltende aliphatische Rest der Esterfunktion des Polymers unter 2-Hexyloctyl-, 2-Octyldecyl- oder 2-Hexyldodecylresten oder Resten, die sich von Isoalkanolen der allgemeinen Formel:
CH₃-CH(CH₃)-(CH₂)ₘ-CH₂OH,
worin m für eine ganze Zahl zwischen 2 und 26 steht, ableiten, und insbesondere unter 4-Methylpentyl-, 5-Methylhexyl-, 6-Methylheptyl-, 15-Methylpentadecyl- oder 16-Methylheptadecylresten ausgewählt ist.

12. Zusammensetzung nach einem der Ansprüche 1 bis 11, bei der 30% bis 98% der Monomereinheiten, die das Polymer enthält, eine freie, teilweise versalzte oder vollständig versalzte starke Säurefunktion aufweisen.

13. Zusammensetzung nach Anspruch 12, bei der das Polymer 30% bis 98% mindestens eines Monomers mit einer starken Säurefunktion, 1% bis 15% mindestens eines Esters mit einer Fettkette einer ungesättigten Carbonsäure, 0,01% bis 5% mindestens eines polyethylenischen Monomers und 0% bis 60% mindestens eines unter neutralen Monomeren oder Monomeren mit nicht veresterter, freier, teilweise versalzter oder vollständig versalzter Carbonsäurefunktion ausgewählten Monomers enthält.

14. Zusammensetzung nach Anspruch 13, bei der das neutrale Monomer des dieses enthaltenden Polymers unter Acrylamid, Methacrylamid, Vinylpyrrolidon, 2-Hydroxyethylacrylat, 2-Hydroxyethylmethacrylat, 2,3-Dihydroxypropylacrylat, 2,3-Dihydroxypropylmethacrylat oder einem ethoxylierten Derivat jedes der oben beschriebenen Hydroxylester mit einem Molekulargewicht zwischen 400 und 1000 ausgewählt ist und das Monomer mit einer nicht veresterten Carbonsäurefunktion unter Acrylsäure, Methacrylsäure, Itaconsäure oder Maleinsäure ausgewählt ist.

## Claims

1. Cosmetic, dermocosmetic, dermopharmaceutical or pharmaceutical, topical composition, **characterized in that** it comprises from 0.1% to 10% by weight of a linear, branched or crosslinked polymer based on at least one monomer having a free, partially salified or completely salified, strong acid function, copolymerized with at least one monomer chosen either from esters of aliphatic alcohols containing from 8 to 30 carbon atoms and of unsaturated monocarboxylic acids, or from esters of aliphatic alcohols containing from 8 to 30 carbon atoms and of unsaturated polycarboxylic acids.

2. Composition as defined in Claim 1, in which said polymer is branched or crosslinked with a crosslinking agent and/or the branching agent chosen from diallyloxyacetic acid or one of the salts, and in particular its sodium salt, trimethylolpropane triacrylate, ethylene glycol dimethacrylate, diethylene glycol diacrylate, tetraethylene glycol diacrylate, methylene-bis(acrylamide), diallyl urea, triallylamine or 1,1,2,2-tetrallyloxyethane.

3. Composition as defined in either of Claims 1 and 2, for which the monomer comprising a strong acid function, of said polymer, is partially or completely salified in the form of an alkali metal salt, such as the sodium salt or the potassium salt, of an ammonium salt (NH₄⁺) or of an aminoalcohol salt, for instance the monoethanolamine salt (HOCH₂CH₂NH₃⁺).

4. Composition as defined in one of Claims 1 to 3, for which the strong acid function of the monomer of said polymer containing it is the sulphonic acid function or the phosphonic acid function.

5. Composition as defined in one of Claims 1 to 4, for which the monomer comprising a strong acid function, of said polymer, is chosen from styrene sulphonic acid, styrene phosphonic acid, 2-sulphoethyl methacrylate or preferably 2-methyl-2-[(1-oxo-2-propenyl)amino]-1-propanesulphonic acid, said acids being free, partially salified or completely salified.

6. Composition as defined in one of Claims 1 to 5, for which the esters of aliphatic alcohols containing from 8 to 30 carbon atoms and of unsaturated monocarboxylic or polycarboxylic acids, of said polymer, are chosen from esters of α-unsaturated acids, and more particularly from α-unsaturated monocarboxylic acids or α-unsaturated dicarboxylic acids.

7. Composition as defined in Claim 6, for which the esters of α-saturated monocarboxylic acids or of α-unsaturated dicarboxylic acids, of said polymer, are chosen from esters of acrylic acid, of methacrylic acid, of itaconic acid or of maleic acid.

8. Composition as defined in one of Claims 1 to 7, for which the aliphatic radical containing from 8 to 30 carbon atoms, of the ester function of said polymer, is a linear or branched, saturated or unsaturated radical.

9. Composition as defined in Claim 8, for which the aliphatic radical containing from 8 to 30 carbon atoms, of the ester function of said polymer, is chosen from radicals derived from linear primary alcohols, and more particularly from octyl, nonyl, decyl, undecyl, 10-undecenyl, dodecyl, tridecyl, tetradecyl, pentadecyl, hexadecyl, heptadecyl, octadecyl, 9-octadecenyl, 10,12-octadecadienyl, 13-docosenyl or triacontanyl radicals.

10. Composition as defined in Claim 8, for which the aliphatic radical containing from 8 to 30 carbon atoms, of the ester function of said polymer, is chosen from radicals derived from the branched 1-alkanols corresponding to the general formula:
CH₃-(CH₂)ₚ-CH[CH₃-(CH₂)ₚ₋₂]-CH₂OH,
in which p represents an integer between 2 and 14, and more particularly from 2-ethylhexyl, 2-propylheptyl, 2-butyloctyl, 2-pentylnonyl, 2-hexyldecyl or 2-octyldodecyl radicals.

11. Composition as defined in Claim 8, for which the aliphatic radical containing from 8 to 30 carbon atoms, of the ester function of said polymer, is chosen from 2-hexyloctyl, 2-octyldecyl or 2-hexyldodecyl radicals, or from radicals derived from the isoalkanols corresponding to the general formula:
CH₃-CH(CH₃)-(CH₂)ₘ-CH₂OH,
in which m represents an integer between 2 and 26, and more particularly from 4-methylpentyl, 5-methylhexyl, 6-methylheptyl, 15-methylpentadecyl or 16-methylheptadecyl radicals.

12. Composition as defined in one of Claims 1 to 11, for which 30% to 98% of the monomeric units that said polymer comprises have a free, partially salified or completely salified, strong acid function.

13. Composition as defined in Claim 12, in which said polymer contains from 30% to 98% of at least one monomer having a strong acid function, from 1% to 15% of at least one ester comprising a fatty chain of an unsaturated carboxylic acid, from 0.01% to 5% of at least one polyethylene monomer, and from 0% to 60% by weight of at least one monomer chosen from neutral monomers or monomers comprising a free, partially salified or completely salified, non-esterified carboxylic acid function.

14. Composition as defined in Claim 13, for which the neutral monomer of said polymer optionally containing it is chosen from acrylamide, methacrylamide, vinylpyrrolidone, 2-hydroxyethyl acrylate, 2-hydroxyethyl methacrylate, 2,3-dihydroxypropyl acrylate, 2,3-dihydroxypropyl methacrylate or an ethoxylated derivative, with a molecular weight of between 400 and 1000, of each of the hydroxylated esters described above, and the monomer comprising a non-esterified carboxylic acid function is chosen from acrylic acid, methacrylic acid, itaconic acid or maleic acid.
